(19) Europäisches Patentamt
European Patent Office
Office européen des brevets

(11) Veröffentlichungsnummer: **0 004 589**
**B1**

# (12) EUROPÄISCHE PATENTSCHRIFT

(45) Veröffentlichungstag der Patentschrift: **10.02.82**

(51) Int. Cl.³: **C 07 C 103/52,** G 01 N 33/48

(21) Anmeldenummer: **79100825.3**

(22) Anmeldetag: **19.03.79**

## (54) Alpha-N-acetyl-L-phenylalanyl-L-arginin-äthylester, Verfahren zu seiner Herstellung und seine Verwendung als Kallikrein-Diagnostikum.

(30) Priorität: **30.03.78 DE 2813772**

(43) Veröffentlichungstag der Anmeldung: **17.10.79 Patentblatt 79/21**

(45) Bekanntmachung des Hinweises auf die Patenterteilung: **10.02.82 Patentblatt 82/6**

(84) Benannte Vertragsstaaten: **BE CH DE FR GB IT NL SE**

(56) Entgegenhaltungen:
**NL-A-64 01 590**
**US-A-3 259 617**

**HELVETICA CHIMICA ACTA, volume 58, 1975, No. 228**
**ALEX EBERLE et al.: «Hormon-Rezeptor-Wechselwirkungen. Synthese von -Melanotropin und von informationstragenden Teilsequenzen unter Verwendung alkalilabiler Schutzgruppen», pages 2106–2129**

**UNLISTED DRUGS, volume 21, February 1969, No. 2 SV 1772, cite: Applied Microbiol. 16:1489, October 1968, «Carbobenzoxy L-phenylalanyl-nitro-L-arginine», page 18e**

**UNLISTED DRUGS, volume 29, January 1977, No. 1 S 2160, cite: R. FED. PROC 26(1) 40, January 1977, «Benzoyl-Phe-Val-Arg-p-nitroaniline», page 86a**

(73) Patentinhaber: **BAYER AG, Zentralbereich Patente, Marken und Lizenzen, D-5090 Leverkusen 1, Bayerwerk (DE)**

(72) Erfinder: **Fiedler, Franz, Dr., Reisingerstrasse 15, D-8000 Muenchen 15 (DE)**

**EP 0 004 589 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# $\alpha$-N-Acetyl-L-phenylalanyl-L-arginin-äthylester, Verfahren zu seiner Herstellung und seine Verwendung als Kallikrein-Diagnostikum

Die vorliegende Erfindung betrifft das neue Dipeptid-Derivat $\alpha$-N-Acetyl-L-phenylalanyl-L-argininäthylester (abgekürzte Schreibweise: Ac-Phe-Arg OEt), ein Verfahren zu seiner Herstellung und seine Säureadditionssalze sowie ihre Verwendung als Kallikrein*-Substrat, vorzugsweise im Rahmen einer diagnostischen Anordnung zur Bestimmung des Kallikrein-Gehalts vorzugsweise kleiner Kallikrein-Mengen, wie sie z.B. im Harn oder Speichel auftreten.

Wegen der Rolle des Kallikreins (EC 3.4.4.21) bei der Regulierung des Blutdrucks oder der Nierenfunktion ist die Kallikrein-Bestimmung im Urin diagnostisch bedeutsam.

Eine verlässliche Bestimmung des Kallikrein-Gehalts ist wegen der geringen Kallikrein-Mengen im Urin mit Hilfe der zur Zeit üblichen Methoden äusserst schwierig durchzuführen. Die bisher bekannten Methoden besitzen folgende Nachteile: Z-Tyr-p-nitrophenylester ist ein empfindliches Substrat für Harn-Kallikrein, es ist jedoch weniger empfindlich als die erfindungsgemässe Verbindung und die Spontanhydrolyse erfolgt sehr rasch.

Weiterhin wurde vor kurzem D-Valyl-leucyl-arginin-p-nitranilid (D-Val-Leu-Arg-p-nitranilid S 2266 KABI, Mölndal) als Substrat für drüsen-Kallikrein eingeführt. Die Bestimmungsmethode mit den erfindungsgemässen Verbindungen ist jedoch 38mal empfindlicher.

Eine sehr empfindliche Bestimmungsmethode für Harn-Kallikrein ist mit Hilfe von radioaktiv markiertem Tos-Arg OMe (Tosylarginimethylester-TAME) (Beaven et al; Clin. Chim. Acta 32, 67–73) möglich. Diese Methode erfordert jedoch eine ganz spezielle und teure Ausrüstung und Einstellung mit einer Standardpräparation von Harn-Kallikrein und erlaubt nicht die kontinuierliche Aufzeichnung der Reaktion.

Demgegenüber ist die Empfindlichkeit der Kallikrein-Bestimmung bei der Verwendung von Ac-Phe-Arg OEt als Substrat bei weitem ausreichend, um eine bequeme und problemlose Bestimmung des Kallikreins beispielsweise im Urin oder im Speichel durchzuführen.

Das Enzym Kallikrein gehört zu der Gruppe der Peptidylpeptid-Hydrolasen und zeichnet sich im Gegensatz zu den meisten Enzymen dieser Klasse durch eine hohe Substratspezifität aus. Es setzt durch Spaltung zweier Peptidbindungen nur aus dem in der Globulinfraktion des Serums vorkommenden und als Kininogen bezeichneten Glykoproteid die pharmakologisch äusserst aktiven Kinine frei.

Die Kallikrein-Aktivität kann anhand seiner blutdrucksenkenden Wirkung am Versuchstier durch Vergleich mit einem Standardpräparat (Padutin®, standardisiertes Kallikrein aus Schweinepankreas) quantitativ bestimmt werden (E. K. Frey et. al.: Das Kallikrein-Kinin-System und seine Inhibitoren, Enke-Verlag, Stuttgart 1968). Diese Methode ist aber relativ ungenau.

Eine weitere biologische Methode zur Bestimmung von Kallikrein erfasst die pro Zeiteinheit freigesetzte Menge an Kinin, die durch die Messung der Uterus- oder Darm-kontrahierenden Wirkung ebenfalls verglichen mit einem Kininstandardpräparat gemessen werden kann (E. K. Frey et. al.: Das Kallikrein-Kinin-System und seine Inhibitoren, Enke-Verlag Stuttgart 1968). Diese Methode ist als biologische Methode ebenfalls relativ ungenau und aufwendig.

Einfacher und exakter als die beiden vorgenannten biologischen Bestimmungsmethoden ist die optische Messung der Aktivität anhand der esterspaltenden Wirkung des Kallikrein.

Als Substrat zur Messung der esterolytischen Aktivität des Kallikreins eignet sich bevorzugt der Benzoyl-L-argininäthyl-ester (Bz-Arg OEt = BAEE). Siehe dazu: Methoden der enzymatischen Analyse Band I, 1071–1080, Weinheim 1974. Das Prinzip dieser Methode kann wie folgt kurz dargestellt werden:

* In vielen Ländern eingetragene Warenzeichen der BAYER AG

$$\text{(A) Bz-Arg OEt} \xrightleftharpoons{\text{Kallikrein}} \text{Bz-Arg} + C_2H_5OH$$

$$\text{(B) } C_2H_5OH + NAD^{\oplus} \xrightleftharpoons{\text{ADH}} CH_3CHO + NADH + H^{\oplus}$$

wobei die in den vorstehenden Formeln gebrauchten Abkürzungen folgende Bedeutung besitzen:

| | | |
|---|---|---|
| Bz-Arg OEt | = | Benzoylargininäthylester |
| Bz-Arg | = | Benzoylarginin |
| $NAD^{\oplus}$ | = | positiv geladene Form des NAD (Nicotinsäureamidadenindinucleotid) |
| ADH | = | Alkoholdehydrogenase (aus Hefe) |
| NADH | = | reduzierte Form des NAD (im Pyridinteil reduziert) |

Das Gleichgewicht (A) liegt weit auf der Seite der Spaltprodukte. Aus der Bz-Arg OEt-Spaltung ergeben sich 5 Möglichkeiten der Aktivitätsbestimmung. Am sichersten und einfachsten ist die enzymatische Messung der freigesetzten Menge an Äthanol in gekoppelter Reaktion (siehe dazu Gleichung (B)). Die pro Zeiteinheit umgesetzte Menge Äthanol, gemessen an der Zunahme der NADH-Extinktion bei einer bestimmten Wellenlänge (z.B. bei 340, 334 oder 365 nm) ist ein Mass für die Kallikrein-Aktivität.

Die Bestimmung des Kallikrein mit Hilfe des erfindungsgemässen Substrats erfolgt in Analogie zum Bz-Arg OEt-Verfahren nach dem folgenden Prinzip:

$$(1)\ \text{Ac-Phe-Arg OEt} \xrightleftharpoons{\text{Kallikrein}} \text{Ac-Phe-Arg} + C_2H_5OH$$

$$(2)\ C_2H_5OH + NAD^{\oplus} \xrightleftharpoons{\text{ADH}} CH_3CHO + NADH + H^{\oplus}$$

(Ac-Phe-Arg: Acetylphenylalanylarginin)

Das Prinzip entspricht also dem von Trautschold und Werle 1961 beschriebenen (Benzoylargininäthylester Bz-Arg OEt = BAEE als Substrat), nur ist die Empfindlichkeit der Bestimmung mit Ac-Phe-Arg OEt um den Faktor 46 grösser. Die nichtenzymatische Konkurrenzreaktionen (z.B. Hydrolyse) sind vergleichbar gering.

Die Reaktion kann kontinuierlich mit einem Spektralphotometer bei z.B. 366 oder 340 nm verfolgt und aufgezeichnet werden.

Die nach Gleichung (2) pro Zeiteinheit umgesetzte Menge Äthanol, gemessen an der Zunahme der NADH-Extinktion, ist ein Mass für die Kallikrein-Aktivität.

Die nach Gleichung (2) entstehende NADH-Menge kann nach folgenden Methoden bestimmt werden:

a) Manuelles Verfahren (UV-Test) mit Spektralphotometer oder Spektrallinienphotometer, Messung der NADH-Extinktion.
b) Bestimmung im Analysenautomaten: gleiches Prinzip wie bei a).
c) Fluorimetrisches Verfahren: das pro Zeiteinheit gebildete NADH (siehe Gleichung (2)) wird nach Zerstörung des NAD-Überschusses durch Behandlung mit starkem Alkali in ein Fluoreszenzprodukt überführt. Die Zunahme der Fluoreszenz pro Zeiteinheit ist ein Mass für die Kallikrein-Aktivität.

Neben den oben aufgeführten biologischpharmakologischen Methoden kann die Aktivitätsbestimmung anhand der esterolytischen Spaltung von Ac-Phe-Arg OEt (gemäss Gleichung (1)) ausserdem nach folgenden bei Bz-Arg OEt bekannten Methoden erfolgen:

1. Kontinuierliche automatische Mikrotitration der freigesetzten Carboxyl-Gruppen (in Analogie zu Tautschold und Werle Hoppe Seylers Z. physiol. Chem. 325, 48 (1961)).
2. Volumetrische Bestimmung der Carboxylgruppen mit Hydrogencarbonat/$CO_2$-Puffer (in Analogie zu der unter 1. genannten Literaturstelle).
3. Bestimmung der Esterbindung als Farb-Test (in Analogie zu S. Hestrin, J. biol. Chem. 180, 249 (1949)).

Im folgenden experimentellen Teil besitzen die dort gebrauchten Abkürzungen bzw. Warenzeichen folgende Bedeutung:

| | |
|---|---|
| Ac-Phe-Arg OEt: | Acetylphenylalanyl-argininäthylester |
| L-Arg OEt·2 HCl: | Hydrochlorid des L-Argininäthylesters (= L-$\alpha$-Amino-$\delta$-guanidyl-valeriansäureäthylester) |
| CM-Sephadex® C-25: | ein mit Carboxymethylgruppen substituiertes quervernetztes Polydextrangel |
| Ac-Phe-Arg | = Acetyl-phenylalanyl-arginin |
| Bz-Arg OEt | = Benzoylargininäthylester |
| NAD | = reduzierte Form des Nicotinsäureamid-adenin-dinucleotids (im Pyridinteil reduziert) |
| Ac-Phe-Arg OEt | = Acetyl-phenylalanyl-argininäthylester |
| ADH | = Alkoholdehydrogenase aus Hefe |
| Phe | = Phenylalanin |
| Arg | = Arginin |
| HOAC | = Essigsäure |

1. Synthese von Ac-Phe-Arg OEt

a) L-Arg OEt · 2 HCl. In Analogie zur Darstellung des entsprechenden Methylesters in R.A. Boissonnas, S. Guttmann, R.L. Huguenin, P.-A. Jaquenond, E. Sandrin, Helv. Chim. Acta, 41, 1867 (1958). 30 ml Äthanol wurden auf –10°C gekühlt und innerhalb von 30 Min. mit 4,75 ml (65 mMol) Thionylchlorid versetzt.

Nach Zusatz von 10,5 g (50 mMol) 1-Arginin. HCL wurde der Ansatz 90 Min. am Rückflusskühler unter Rühren und Feuchtigkeitsausschluss auf 45°C gehalten und dann über Nacht bei Zimmertemperatur weitergerührt. Es fielen 8,0 g (58%) des Esters aus. Umkristallisation aus Äthanol. Fp. (unkorr.) 119–120°C. G. Weitzel, R. Renner, H. Guglielmi, Hoppe-Seyler's Z. Physiol., Chem. 352, 1617 (1971) geben. Fp. 115°C an (Synthese mit Äthanol/HCl).

$[\alpha]^{27°C}_D = +11{,}6° \pm 0{,}3°$ (c = 2 in Wasser).

Analyse:

| | | | |
|---|---|---|---|
| Berechnet | C 34,92 | H 7,33 | N 20,36% |
| Gefunden | C 34,90 | H 7,24 | N 20,70% |

Das Produkt ist dünnschichtchromatographisch (Kieselgel; Laufmittel n-Butanol-Eisessig-Wasser 80:20:20 und n-Butanol-Pyridin-Eisessig-Wasser 40:10:10:20; Anfärbung mit Sakaguchi-Reagenz und mit Chlor-Tolidin) selbst bei Auftragung von 200 mMol einheitlich.

b) Ac-Phe-Arg OEt · HOAC

0,829 g (4 mMol) N-Acetyl-L-phenylalanin wurden in 40 ml Dimethylformamid gelöst.

1,10 g (4 mMol) fein pulverisiertes L-Argininäthylester-dihydrochlorid, 0,56 ml (4 mMol) Triäthylamin und 0,69 g (6 mMol) N-Hydroxysuccinimid wurden zugesetzt und der Ansatz auf –15 °C abgekühlt. Nach Zusatz von 0,91 g (4,4 mMol) Dicyclohexylcarbodiimid wurde 7 h bei –5 °C, über Nacht bei 5 °C und 5 h bei Zimmertemperatur gerührt. Der Niederschlag wurde abfiltriert und die Lösung im Vakuum am Rotationsverdampfer bis fast zur Trockene gebracht. Nach Aufnehmen in 80 ml Wasser wurde der pH mit Triäthylamin auf 6,0 eingestellt und die Lösung auf eine Säule (1,5 × 40 cm) mit CM-Sephadex®C-25, äquilibriert mit 0,05 M Triäthylammoniumacetat pH 6,0, aufgetragen. Eluiert wurde mit einem linear auf 0,2 M Triäthylammoniumacetat, pH 6,0, ansteigenden Gradienten (720 ml; 50 ml/h; Fraktionsgrösse 8 ml). Die Fraktionen 75–110, die nach Tüpfelung auf Filterpapier und Ansprühen mit Sakaguchi-Reagenz die Hauptmenge des eluierten Materials enthielten, wurden lyophilisiert. Die vereinigten Rückstände wurden mit 0,5 ml Äthanol versetzt und mit 60 ml Essigsäureäthylester gefällt. Der Niederschlag wurde im Exsikkator über Phosphorpentoxid getrocknet. Ausbeute 1,28 g ≙ 71%. Fp. (unkorr.) 81–85 °C.

$[\alpha]^{27°}_D = -9,8° \pm 0,3°$ (c = 2 in Wasser).

Analyse:

| | | | |
|---|---|---|---|
| Berechnet | C 55,86 | H 7,37 | N 15,51%. |
| Gefunden | C 55,57 | H 7,58 | N 15,08%. |

Dünnschichtchromatographisch unter obigen Bedingungen einheitlich; nach Hydrolyse mit Rindertrypsin war nur Ac-Phe-Arg nachzuweisen. Titration mit Lauge nach Hydrolyse des Esters mit Rindertrypsin: 100,2% der Theorie. Aminosäureanalyse nach HCl-Hydrolyse: $Phe_1$ $Arg_{0,990}$; Gehalt an Aminosäuren: 102,6% der Theorie.

2. Bestimmung von menschlichem Harn-Kallikrein mit Ac-Phe-Arg OEt

(in Anlehnung an den Test mit Bz-Arg OEt von I. Trautschold und E. Werle, Hoppe-Seyler's Z. Physiol. Chem. 325 (1961) 48-59).

In einer Küvette mit 1 cm Schichtdicke, thermostatisiert auf 25,0 °C, werden gemischt:

2,00 ml Puffer (0,15 M Natriumpyrophosphat, 0,15 M Semicarbazidhydrochlorid, 0,0375 M Glycin; pH mit Natronlauge auf 8,7 eingestellt)
0,10 ml NAD (30 mM in Wasser)
0,10 ml Ac-Phe-Arg OEt (als Acetat; 15 mM in Wasser)
0,020 ml ADH (Alkoholdehydrogenase aus Hefe, Boehringer; Suspension mit 100 mg/3,4 ml)

Wasser zur Einstellung des Endvolumens (nach Probenzusatz) auf 3,00 ml. Der Ansatz wird 5 Min. vorinkubiert. Dann wird die Reaktion durch Zusatz von 0,010 bis 0,500 ml Probe (Lösung von gereinigtem menschlichem Harn-Kallikrein in Wasser) gestartet. Die Menge des Enzyms ist so zu wählen, dass die Extinktionszunahme in 10 Min. zwischen 0,05 und 0,2 Extinktionseinheiten liegt. Bis zu letzterem Wert ist die Reaktionsgeschwindigkeit der Menge an Harn-Kallikrein proportional. Beginnend 2 Min. nach Zusatz der Probe wird die Extinktionszunahme bei 366 nm gegen eine Vergleichsküvette mit Wasser über genau 10 Min. hinweg verfolgt. Parallel dazu wird ein Blindwert mitgeführt, bei dem die Harn-Kallikreinprobe durch Wasser ersetzt ist (pro Arbeitstag genügt die Ermittlung von 2 bis 3 Blindwerten). Aus der gemessenen Extinktionszunahme der Probe wird nach Abzug des Blindwertes mit Hilfe des Extinktionskoeffizienten von NADH die Menge des zugesetzten Harn-Kallikreins in U (1U entspricht der Hydrolyse von 1 μMol Ac-Phe-Arg OEt pro Min. unter den obigen Bedingungen) berechnet (pro Mol hydrolysiertem Ester wird 1 Mol Äthanol freigesetzt, das 1 Mol NADH liefert).

## Patentansprüche

1. Acetylphenylalanyl-arginin-äthylester (Ac-Phe-Arg OEt) und seine Säureadditionssalze.

2. Verfahren zur Herstellung des Acetylphenylalanyl-argininäthylesters, dadurch gekennzeichnet, dass man N-Acetyl-L-phenylalanin in einem organischen Lösungsmittel in Gegenwart von Trialkylamin und einem wasserbindenden Reagenz mit L-Argininäthylester bzw. seinem Hydrochlorid umsetzt und das Reaktionsprodukt nach an sich bekannten Methoden isoliert.

3. Verfahren gemäss Anspruch 2, dadurch gekennzeichnet, dass man N-Acetyl-L-phenylalanin in Dimethylformamid mit L-Argininäthylester-dihydrochlorid in Gegenwart von Triäthylamin und N-Hydroxysuccinimid umsetzt und das Reaktionsprodukt durch Chromatographie an einem Carboxymethylgruppen-haltigen quervernetzten Polydextrangel und anschliessende Lösung in Äthanol und Fällung mit Essigsäureäthylester gewinnt.

4. Verwendung von Acetylphenylalanyl-arginin-äthylester (Ac-Phe-Arg OEt) als Substrat für Kallikrein bei der Bestimmung kleiner Mengen Kallikrein.

5. Verwendung von Ac-Phe-Arg OEt gemäss Anspruch 4 als Substrat für Kallikrein aus Harn.

6. Verwendung von Ac-Phe-Arg OEt gemäss Anspruch 4 als Substrat für Kallikrein aus Speichel.

7. Verwendung von Ac-Phe-Arg OEt gemäss Anspruch 4 als Substrat für Kallikrein aus Drüsen.

## Claims

1. Acetylphenylalanyl-arginine ethyl ester (Ac-Phe-Arg OEt) and its acid addition salts.

2. Process for the preparation of acetylphenylanyl-arginine ethyl ester, characterised in that N-acetyl-L-phenylalanine is reacted with L-arginine ethyl ester, or its hydrochloride, in an organic solvent in the presence of trialkylamine and a water-binding reagent and the reaction product is

isolated by methods which are in themselves known.

3. Process according to Claim 2, characterised in that N-acetyl-L-phenylalanine in dimethylformamide is reacted with L-arginine ethyl ester dihydrochloride in the presence of triethylamine and N-hydroxysuccinimide and the reaction product is isolated by chromatography on a crosslinked polydextran gel containing carboxymethyl groups and subsequent solution in ethanol and precipitation with ethyl acetate.

4. Use of acetylphenylalanyl-arginine ethyl ester (Ac-Phe-Arg OEt) as a substrate for Kallikrein in the determination of small amounts of Kallikrein.

5. Use of Ac-Phe-Arg OEt according to Claim 4 as a substrate for Kallikrein from urine.

6. Use of Ac-Phe-Arg OEt according to Claim 4 as a substrate for Kallikrein from saliva.

7. Use of Ac-Phe-Arg OEt according to Claim 4 as a substrate for Kallikrein from glands.

**Revendications**

1. Ester éthylique d'acétylphénylalanyl-arginine (Ac-Phe-Arg-OEt) et ses sels d'addition d'acides.

2. Procédé pour la préparation de l'ester éthylique d'acétylphénylalanyl-arginine, caractérisé en ce que l'on fait réagir la N-acétyl-L-phénylalanin avec l'ester éthylique de L-arginine ou son chlorhydrate dans un solvant organique en présence de trialkylamine et d'un réactif fixant l'eau et on isole le produit de réaction par des techniques connues.

3. Procédé selon la revendication 2, caractérisé en ce que l'on fait réagir la N-acétyl-L-phénylalanine dans le diméthyl-formamide avec le dichlorhydrate d'ester éthylique de L-arginine en présence de triéthylamine et de N-hydroxysuccinimide et on récupère le produit de réaction par chromatographie sur un gel de polydextranne réticulé contenant des groupes carboxyméthyle et ensuite dissolution dans l'éthanol et précipitation par l'acétate d'éthyle.

4. Utilisation de l'ester éthylique d'acétylphénylalanyl-arginine (Ac-Phe-Arg-OEt) comme substrat pour la kallikréine dans la détermination de faibles quantités de kallikréine.

5. Utilisation de Ac-Phe-Arg-OEt selon la revendication 4 comme substrat pour la kallikréine de l'urine.

6. Utilisation de Ac-Phe-Arg-OEt selon la revendication 4 comme substrat pour la kallikréine de la salive.

7. Utilisation de Ac-Phe-Arg-OEt selon la revendication 4 comme substrat pour la kallikréine des glandes.